# EUROPÄISCHE PATENTANMELDUNG

(11) **EP 4 316 563 A2**
(43) Veröffentlichungstag der Anmeldung: **07.02.2024**
(21) Anmeldenummer: 23188631.8
(22) Anmeldetag: 31.07.2023
(51) Int. Cl.: A61M 39/04, A61M 39/18, A61M 39/22, A61M 39/26, A61M 39/06, A61M 39/10, A61M 39/24

(54) **VERFAHREN, VORRICHTUNGEN UND MEDIZINPRODUKT**

(30) Priorität: 01.08.2022 DE 102022207916
(71) Anmelder: B. Braun Melsungen AG, 34212 Melsungen (DE)
(72) Erfinder: Kaeufer, Guido, 4557 Horriwil (CH); Schneider, Martin, 3604 Thun (CH); Dudhane, Mayur Suresh, 6170 Schüpfheim (CH); Gläß, Liesa, 6170 Schüpfheim (CH); Roth, Norbert, 6170 Schüpfheim (CH); Andree, Robert, 34119 Kassel (DE)
(74) Vertreter: Patentanwälte Ruff, Wilhelm, Beier, Dauster & Partner mbB

(57) **Zusammenfassung**

Die Erfindung betrifft ein Verfahren zum Herstellen eines elastisch deformierbaren Funktionsteils für ein Medizinprodukt, wobei das Funktionsteil eine öffnungsfähige Schlitzanordnung aufweist, die sich bei einer elastischen Deformation des Funktionsteils aufweitet oder öffnet und bei Wegfall der Deformation wieder schließt. Die Erfindung betrifft zudem Vorrichtungen für derartige Verfahren und ein Medizinprodukt mit einem solchen Funktionsteil.

## Beschreibung

Die Erfindung betrifft mit einem ersten Aspekt ein Verfahren zum Herstellen eines elastisch deformierbaren Funktionsteils, insbesondere Funktionsteil-Rohlings, für ein Medizinprodukt. Mit einem zweiten Aspekt betrifft die Erfindung ein weiteres Verfahren zum Herstellen eines elastisch deformierbaren Funktionsteils für ein Medizinprodukt. Darüber hinaus betrifft die Erfindung mit einem dritten Aspekt eine Vorrichtung für die Herstellung eines elastisch deformierbaren Funktionsteils, insbesondere eines Funktionsteil-Rohlings, für ein Medizinprodukt. Zudem betrifft die Erfindung mit einem vierten Aspekt eine weitere Vorrichtung für die Herstellung eines elastisch deformierbaren Funktionsteils für ein Medizinprodukt. Schließlich betrifft die Erfindung mit einem fünften Aspekt ein derartiges Medizinprodukt, insbesondere eine Silikonventileinrichtung.

Gamma-Bestrahlung stellt ein gängiges Sterilisationsverfahren für Medizinprodukte wie Silikonventileinrichtungen dar. Weisen die Medizinprodukte ein strahlenvernetzbares Material - wie beispielsweise Silikonelastomere - auf, reagieren diese Materialien bei Einwirkung energiereicher Strahlung mit Vernetzungsreaktionen. Im Falle von als Schlitzventilen ausgebildeten Silikonventileinrichtungen kann dies zu einem unerwünschten Zuwachsen der Schlitze, auch als Heilen bezeichnet, führen.

Um ein solches Heilen von Schlitzen zu verhindern, werden üblicherweise Additive, sogenannte Trennmittel, zugesetzt. Die Trennmittel können in fester Form, beispielsweise in Form von Kunststoffpartikeln, verwendet werden. Nachteilig ist, dass eine solche Additivierung in der Regel zu opaken Materialien führt. Für viele Anwendungen, beispielsweise Verbindungseinrichtungen im Bereich der medizinischen Infusionstherapie, ist jedoch der Erhalt von Transparenz einzelner Funktionsteile, wie beispielsweise Ventilkörpern von Schlitzventilen, für den bestimmungsgemäßen Gebrauch unbedingt erforderlich. Ferner ist problematisch, dass der Zusatz fester Trennmittel grundlegende Änderungen von Materialeigenschaften bewirken kann.

Schlitze von derartigen Funktionsteilen werden üblicherweise durch ein Durchtrennen eines Materials eines Rohlings für das Funktionsteil mittels einer metallenen Klinge erzeugt. Diese verschleißt aber mit der Zeit und wird entsprechend stumpf. Eine verschlissene, abgestumpfte Klinge muss durch eine scharfe Klinge ersetzt werden. Dies kostet Zeit und Geld.

Neben festen Trennmitteln kommen auch flüssige Trennmittel zum Einsatz. Diese müssen jedoch eine ausreichende Benetzbarkeit und insbesondere Ortsbeständigkeit aufweisen, um ihre Funktion, insbesondere dauerhaft, ausüben zu können.

Strahlensterilisation, Alterungsprozesse sowie Transportvorgänge bringen in der Regel erhöhte Temperaturen mit sich, wodurch sich die Trennwirkung flüssiger Trennmittel über die Zeit häufig als zu gering erweist. Hinzu kommt, dass manche flüssige Trennmittel, wie beispielsweise Silikonöle, ebenfalls auf eine Strahlensterilisation reagieren, wodurch die Trennwirkung zusätzlich geschmälert werden kann. Das Aufbringen eines derartigen Trennmittels an dem geschlitzten Funktionsteil erweist sich als herausfordernd.

### AUFGABE UND LÖSUNG

Der vorliegenden Erfindung liegt daher die Aufgabe zugrunde, Verfahren zum Herstellen eines elastisch deformierbaren Funktionsteils für ein Medizinprodukt, Vorrichtungen für die Herstellung eines derartigen Funktionsteils sowie ein derartiges Medizinprodukt bereitzustellen, die gegenüber herkömmlichen Herstellungsverfahren und Vorrichtungen für solche Herstellungsverfahren und entsprechenden Medizinprodukten verbesserte Eigenschaften aufweisen. Insbesondere soll eine besonders wartungsarme und damit einhergehend kostengünstige Herstellung derartiger elastisch deformierbarer Funktionsteile ermöglicht werden.

Diese Aufgabe wird gelöst durch ein Verfahren gemäß dem unabhängigen Anspruch 1, durch ein Verfahren gemäß dem unabhängigen Anspruch 7 sowie durch eine Vorrichtung gemäß dem nebengeordneten Anspruch 14, eine Vorrichtung gemäß dem nebengeordneten Anspruch 15 und durch ein Medizinprodukt gemäß dem nebengeordneten Anspruch 16. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung ein Verfahren zum Herstellen eines elastisch deformierbaren Funktionsteils, insbesondere Funktionsteil-Rohlings, für ein Medizinprodukt, wobei das Funktionsteil eine öffnungsfähige Schlitzanordnung aufweist, die sich bei einer elastischen Deformation des Funktionsteils aufweitet oder öffnet und bei Wegfall der Deformation wieder schließt. Das Verfahren umfasst einen Schritt a), gemäß welchem ein Bereitstellen eines Rohlings für das Funktionsteil, der ein elastisches, und insbesondere transparentes, Material aufweist oder aus einem elastischen, und insbesondere transparenten, Material besteht, erfolgt. Das Verfahren umfasst zudem einen Schritt b), gemäß welchem ein wenigstens bereichsweises, insbesondere entlang einer Dicke vollständiges, Durchtrennen des Materials des Rohlings mittels Ultraschalls erfolgt, so dass wenigstens ein Schlitz der Schlitzanordnung des Funktionsteils erzeugt wird. Im geöffneten oder aufgeweiteten Zustand ist die Schlitzanordnung vorzugsweise fluiddurchlässig. Im deformationsfreien, geschlossenen Zustand der Schlitzanordnung ist sie vorzugsweise fluiddicht. Vorteilhaft kann durch Verwendung von Ultraschall auf eine Klinge zum Erzeugen des Schlitzes verzichtet werden. Derartige Klingen unterliegen mechanischem Verschleiß, der zu einem Abstumpfen einer derartigen Klinge über die Nutzungszeit führt. Mittels Ultraschalls lässt sich das Material des Rohlings verschleißarm oder sogar verschleißfrei schlitzen. Somit können einerseits die Kosten für einen herkömmlicherweise wiederkehrend erforderlichen Klingenersatz eingespart werden und andererseits auch die Zeit, die für einen entsprechenden Austausch einer derartigen Klinge anfällt. Zudem weisen Schlitzwandungsflächen eines mittels Ultraschalls erzeugten Schlitzes besonders gute Hafteigenschaften für ein etwaiges, auf Schlitzwandungsflächen aufbringbares Hemm-Material auf, welches eine Selbstheilung des Materials des Funktionsteils hemmen oder verhindern kann.

Unter dem Ausdruck "Funktionsteil" soll im Sinne der vorliegenden Erfindung eine Komponente oder ein Bauteil oder ein Bauteilelement des Medizinprodukts verstanden werden. Insbesondere kann es sich bei einem derartigen Funktionsteil um einen Ventilkörper für ein als Silikonventileinrichtung, insbesondere Schlitzventileinrichtung, ausgebildetes Medizinprodukt handeln.

Unter dem Ausdruck "Medizinprodukt" soll im Sinne der vorliegenden Erfindung insbesondere ein medizintechnisches Produkt wie eine Silikonventileinrichtung, vorzugsweise eine Schlitzventileinrichtung, beispielsweise für den Gebrauch in medizintechnischer oder medizinischer Anwendung, so für eine Therapie eines Patienten, verstanden werden.

Unter dem Ausdruck "Schlitzwandungsflächen" sollen im Sinne der vorliegenden Erfindung Flächen oder Oberflächen von Wandungen, die einen Schlitz bilden oder definieren oder an der Ausbildung eines Schlitzes beteiligt sind, verstanden werden.

Unter dem Ausdruck "Trennmittel" soll im Sinne der vorliegenden Erfindung eine Substanz oder eine Zusammensetzung verstanden werden, die in der Lage ist, ein Zuwachsen, insbesondere strahleninduziertes Zuwachsen, von Schlitzen zu reduzieren oder sogar vollständig zu vermeiden. Unter "Zuwachsen" kann ein Vernetzen verstanden werden. Der Begriff "Hemm-Material" wird dabei im Zusammenhang mit der vorliegenden Erfindung synonym zum "Trennmittel" verwendet.

Unter dem Ausdruck "transparent" soll im Sinne der vorliegenden Erfindung die Fähigkeit von Materie verstanden werden, elektromagnetische Wellen mit einer Wellenlänge von 380 nm bis 780 nm hindurchzulassen (Transmission). Insofern soll der Begriff "transparent" synonym zu "durchlässig für sichtbares Licht" verstanden werden. Die Transparenz des elastisch deformierbaren Funktionsteils bleibt auch bei Deformation vorteilhafterweise wenigstens teilweise, insbesondere vollständig, erhalten. Es ist auch denkbar, dass das Material des Funktionsteils gefärbt oder getönt ausgebildet ist, so dass es lediglich für Licht mit einer oder mehreren Farben durchlässig ist, wohingegen Licht mit davon abweichenden Wellenlängen das Material nicht durchdringen kann.

Zweckmäßig umfasst die Schlitzanordnung einen oder mehrere Schlitze. Die Schlitzanordnung kann mehrere Schlitze in Kreuz- oder Sternanordnung aufweisen. Eine solche Mehrzahl an Schlitzen kann simultan oder nacheinander erzeugt werden. Ein derartiger Schlitz kann geradlinig oder gekrümmt geformt werden.

In Ausgestaltung des Verfahrens nach dem ersten Aspekt der Erfindung umfasst der Schritt b) einen Unterschritt b1), gemäß welchem ein Aktivieren einer Sonotrode erfolgt, so dass die Sonotrode in eine Schwingung mit Ultraschallfrequenz versetzt wird. Die Sonotrode kann von einem Ultraschallgenerator gespeist werden, um in Ultraschallschwingung versetzt zu werden. Die Ultraschallfrequenz kann 20 kHz bis 40 kHz, vorzugsweise 30 kHz bis 38 kHz, betragen. Mittels einer derartigen Sonotrode lässt sich der Schlitz besonders positionsgetreu anbringen. Somit lassen sich besonders eng tolerierte Funktionsteile, insbesondere Funktionsteil-Rohlinge, fertigen. Die positionsgetreue Anbringung des Schlitzes kann durch eine präzise Führung der Sonotrode, insbesondere einer die Sonotrode umfassenden Ultraschalleinheit, begünstigt werden.

Zweckmäßig schwingt die aktivierte Sonotrode längs und/oder quer zu ihrer Erstreckung, d. h. eine Amplitude der Ultraschallschwingung ist entlang und/oder quer zur Erstreckung der Sonotrode messbar. Bevorzugt schwingt die aktivierte Sonotrode längs und/oder quer zur Schlitzrichtung, d. h. eine Amplitude der Ultraschallschwingung ist entlang und/oder quer zur Schlitzrichtung messbar.

In weiterer Ausgestaltung des Verfahrens nach dem ersten Aspekt der Erfindung umfasst der Schritt b) einen Unterschritt b2), gemäß welchem ein Verstellen der Sonotrode und/oder des Rohlings relativ zueinander in einer Schlitzrichtung erfolgt, um das Material des Rohlings wenigstens bereichsweise zu durchtrennen und den wenigstens einen Schlitz zu erzeugen. Zweckmäßig erfolgt das Verstellen gemäß Unterschritt b2) mit einer Vorschubgeschwindigkeit von 0,02 m/s bis 0,2 m/s, vorzugsweise 0,05 m/s bis 0,15 m/s. Die Vorschubgeschwindigkeit kann während des Verstellens variiert werden. Die Vorschubgeschwindigkeit kann während des Schlitz- oder Schneidvorgangs zur Erzeugung des Schlitzes variiert werden. Insbesondere kann die Vorschubgeschwindigkeit mittels eines geschlossenen Regelkreises in Abhängigkeit zu einer dem Vorschub entgegengesetzten Schneidkraft so geregelt werden, dass das Funktionsteil während der Erzeugung des Schlitzes nicht deformiert wird. Auf diese Weise lässt sich der Schlitz besonders formgetreu erzeugen. Ferner lässt sich auf diese Weise ein Schlitz erzeugen, an dessen Schlitzwandungsflächen ein etwaiges Hemm-Material besonders gut haften kann. Zudem kann auf diese Weise eine im deformationsfreien Zustand besonders fluiddichte Schlitzanordnung erzeugt werden. Das Funktionsteil kann im montierten Zustand unter Vorspannung stehen, die mit der Zeit infolge von Kriechverhalten nachlassen kann.

In weiterer Ausgestaltung des Verfahrens nach dem ersten Aspekt der Erfindung umfasst der Schritt b) einen Unterschritt b3), gemäß welchem ein Verweilen der Sonotrode in einer vorbestimmten Endposition für eine vorbestimmte Verweildauer erfolgt, wenn die Sonotrode bei Durchführung des Unterschritts b2) die vorbestimmte Endposition relativ zum Rohling erreicht hat. Hierdurch lässt sich vorteilhaft verhindern, dass die noch frischen Schlitzwandungsflächen direkt nach ihrer Erzeugung wieder in gegenseitige Anlage gebracht werden, was für die Selbstheilung förderlich wäre. Die Verweildauer kann 50 ms bis 500 ms, vorzugsweise 80 ms bis 300 ms, betragen. Vorzugsweise durchragt die Sonotrode den Schlitz in der Endposition vollständig.

Zweckmäßig lässt sich eine Schnittqualität bei der Erzeugung des Schlitzes durch die Wahl von Verfahrensparametern wie der Ultraschallfrequenz und/oder der Vorschubgeschwindigkeit und/oder der Verweildauer gezielt beeinflussen, insbesondere um eine Fluiddichtheit der Schlitzanordnung und/oder eine Haftung eines etwaigen Hemm-Materials an den Schlitzwandungsflächen zu verbessern. Die Schnittqualität kann dabei eine Abweichung der erzeugten Gestalt des Schlitzes von einer vorbestimmten Soll-Gestalt ausdrücken.

In weiterer Ausgestaltung des Verfahrens nach dem ersten Aspekt der Erfindung wird zum Abschluss der Durchführung des Schritts b) die Sonotrode deaktiviert, so dass die Schwingung der Sonotrode angehalten wird. Die Sonotrode hört dann also auf zu schwingen. Die Sonotrode kann vor, während oder nach dem Verweilen in der Endposition deaktiviert werden. Durch das Verweilen in der Endposition lässt sich sicherstellen, dass das Material des Funktionsteils vollständig durchtrennt wird. Umgekehrt kann das Funktionsteil unter Umständen unbrauchbar werden, wenn die Verweildauer zu lang ist.

Zweckmäßig schwingt die aktivierte Sonotrode mit 50 % bis 100 %, vorzugsweise 60 % bis 90 %, einer Amplitudenleistung. Ist die Sonotrode deaktiviert, so ist keine Amplitude mehr messbar.

In weiterer Ausgestaltung des Verfahrens nach dem ersten Aspekt der Erfindung umfasst das Verfahren einen zusätzlichen Schritt c), gemäß welchem ein Entfernen der Sonotrode und/oder des geschlitzten Funktionsteils voneinander erfolgt. Vorzugsweise ist die Sonotrode beim Entfernen gemäß Schritt c) bereits deaktiviert. Hierdurch lässt sich vorteilhaft einer Selbstheilung entgegenwirken oder eine derartige Selbstheilung wenigstens verzögern. Zudem kann auf diese Weise vermieden werden, dass Schlitzwandungsflächen des erzeugten Schlitzes beim Entfernen der Sonotrode beschädigt werden.

Gemäß einem zweiten Aspekt betrifft die Erfindung ein weiteres Verfahren zum Herstellen eines elastisch deformierbaren Funktionsteils für ein Medizinprodukt, wobei das Funktionsteil eine öffnungsfähige Schlitzanordnung aufweist, die sich bei einer elastischen Deformation des Funktionsteils aufweitet oder öffnet und bei Wegfall der Deformation wieder schließt. Das weitere Verfahren umfasst einen Schritt A), gemäß welchem ein Bereitstellen eines geschlitzten Funktionsteil-Rohlings mit einer Schlitzanordnung erfolgt, die wenigstens einen Schlitz aufweist, an welchem ein elastisches, und insbesondere transparentes, Material des Funktionsteil-Rohlings wenigstens bereichsweise, bevorzugt mittels Ultraschalls, durchtrennt ist. Vorzugsweise ist der wenigstens eine Schlitz gemäß dem Verfahren nach dem ersten Aspekt der Erfindung wie voranstehend beschrieben erzeugt. Mit anderen Worten: Bei dem Funktionsteil-Rohling für das Verfahren nach dem zweiten Erfindungsaspekt kann das mittels des Verfahrens nach dem ersten Erfindungsaspekt hergestellte Funktionsteil, insbesondere Funktionsteil-Rohling, verwendet werden. Entsprechend können die Verfahren nach den ersten beiden Erfindungsaspekten nacheinander im Zuge eines einzigen Durchführungsvorgangs durchgeführt werden. Das Verfahren nach dem zweiten Erfindungsaspekt umfasst außerdem einen Schritt B), gemäß welchem ein Aufweiten oder Öffnen der Schlitzanordnung erfolgt. Zudem weist das Verfahren nach dem zweiten Erfindungsaspekt einen Schritt C) auf, gemäß welchem ein wenigstens bereichsweises, vorzugsweise vollflächiges, Versehen wenigstens einer Schlitzwandungsfläche der Schlitzanordnung des Funktionsteil-Rohlings mit einem Hemm-Material erfolgt, das eine Selbstheilung des Materials des Funktionsteil-Rohlings, insbesondere im Bereich seiner Schlitzanordnung, hemmt oder verhindert. Zweckmäßig kann das Versehen der Schlitzwandungsfläche gemäß Schritt C) durch ein Beschichten oder Beimpfen der betreffenden Schlitzwandungsfläche erfolgen. Vorzugsweise werden zwei komplementäre, höchst vorzugsweise alle, Schlitzwandungsflächen der Schlitzanordnung mit dem Hemm-Material versehen. Hierdurch lässt sich vorteilhaft eine Öffnungsfähigkeit der Schlitzanordnung, insbesondere dauerhaft, gewährleisten. Da der Schlitz des Funktionsteil-Rohlings, bevorzugt mittels Ultraschalls, durchtrennt ist, kann das Hemm-Material an den Schlitzwandungsflächen besonders gut haften.

In Ausgestaltung des Verfahrens nach dem zweiten Aspekt der Erfindung umfasst der Schritt B) einen Unterschritt B1), gemäß welchem ein Einspannen des geschlitzten Funktionsteil-Rohlings, insbesondere mittels einer Spannvorrichtung, erfolgt. Der Schritt B) umfasst zudem einen Unterschritt B2), gemäß welchem ein Deformieren des Funktionsteil-Rohlings erfolgt, um die Schlitzanordnung des Funktionsteil-Rohlings zu öffnen oder aufzuweiten. Dies verbessert vorteilhafterweise die Zugänglichkeit der wenigstens einen Schlitzwandungsfläche für das Versehen derselben mit dem Hemm-Material.

In weiterer Ausgestaltung des Verfahrens nach dem zweiten Aspekt der Erfindung wird zeitlich vor Durchführung des Unterschritts B1) und/oder des Unterschritts B2) der Funktionsteil-Rohling, insbesondere deformationsfrei, an oder auf einem Dorn angeordnet. Alternativ oder zusätzlich werden während der Durchführung des Unterschritts B2) der Dorn und/oder der Funktionsteil-Rohling relativ zueinander verstellt, so dass der Funktionsteil-Rohling mittels des Dorns deformiert wird. Vorzugsweise drückt der Dorn zum Deformieren des Funktionsteil-Rohlings entlang einer axialen Erstreckung des Dorns gegen einen Bereich des Funktionsteil-Rohlings, in welchem die Schlitzanordnung vorhanden ist. Der Dorn kann also gegen die Schlitzanordnung drücken, um diese aufzuweiten oder zu öffnen. Die mittels des Dorns aufgeweitete oder geöffnete Schlitzanordnung kann einen aufklaffenden Schlitz aufweisen, dessen Schlitzwandungsflächen winkelig gegeneinander angeordnet sind, so dass sich der Schlitz vom Dorn abgewandt aufweitet oder öffnet und sich dem Dorn zugewandt verjüngt. Hierdurch ergibt sich eine besonders gute Zugänglichkeit der wenigstens einen Schlitzwandungsfläche für ihr Versehen mit dem Hemm-Material.

Zweckmäßig wird der Dorn in eine Bohrung des Funktionsteil-Rohlings eingeführt, die einenends für die Aufnahme des Dorns geöffnet und anderenends von einem integralen Membranabschnitt des Funktionsteil-Rohlings verschlossen ist, wobei die Schlitzanordnung an dem Membranabschnitt vorhanden ist. Hierdurch lässt sich die Deformation des Funktionsteil-Rohlings besonders gut auf das Versehen der Schlitzwandungsflächen mit dem Hemm-Material anpassen. Zudem kann der Dorn als Positionierhilfe für die Ausrichtung des Funktionsteil-Rohlings dienen, sodass das Hemm-Material besonders präzise am bestimmungsgemäßen Ort anbringbar ist. Der Dorn kann die Schlitzanordnung in Schlitzrichtung abdichten, um zu verhindern, dass Hemm-Material eine Innenseite des Funktionsteils und/oder eine Vorrichtung für die Herstellung des Funktionsteils kontaminiert.

In weiterer Ausgestaltung des Verfahrens nach dem zweiten Aspekt der Erfindung umfasst der Schritt C) einen Unterschritt C1), gemäß welchem ein gegenseitiges Annähern des Funktionsteil-Rohlings und einer Dosiereinheit zum Austragen des Hemm-Materials erfolgt, bis ein vorbestimmter Soll-Auftragsabstand erreicht wird. Hierzu können der Funktionsteil-Rohling und/oder die Dosiereinheit relativ zueinander verstellt werden. Der Soll-Auftragsabstand kann null sein. Der Soll-Auftragsabstand kann kleiner null sein, d. h. die Dosiereinheit kann in diesem Fall teilweise in die Schlitzanordnung hineinragen oder eintauchen. Der Soll-Auftragsabstand kann - 0,4 mm bis + 0,2 mm, vorzugsweise - 0,2 mm bis + 0,1 mm, betragen. Der Schritt C) umfasst zudem einen Unterschritt C2), gemäß welchem - wenn der Soll-Auftragsabstand erreicht ist - ein Austragen des Hemm-Materials erfolgt, um das Hemm-Material wenigstens bereichsweise, vorzugsweise vollflächig, auf die wenigstens eine Schlitzwandungsfläche, vorzugsweise auf zwei komplementäre, höchst vorzugsweise alle Schlitzwandungsflächen, aufzutragen. Damit kann der Selbstheilung besonders wirksam entgegengewirkt werden.

In weiterer Ausgestaltung des Verfahrens nach dem zweiten Aspekt der Erfindung umfasst das Verfahren einen zusätzlichen Schritt D), gemäß welchem ein wenigstens teilweises Entfernen eines überschüssigen Volumens des Hemm-Materials erfolgt, um das überschüssige Volumen von einem die wenigstens eine Schlitzwandungsfläche wenigstens bereichsweise benetzenden Auftragsvolumen des Hemm-Materials abzutrennen. Das Abtrennen des überschüssigen Volumens von dem Auftragsvolumen kann durch Umkehr einer Pumprichtung oder Extrusionsrichtung oder Förderrichtung der Dosiereinheit und/oder durch Verstellen derselben relativ zum Funktionsteil-Rohling erfolgen. Hierdurch lässt sich vorteilhaft ein teilweises "Zurückziehen" einer ausgetragenen Menge des Hemm-Materials, insbesondere einer Raupe des Hemm-Materials, erreichen. Alternativ oder zusätzlich kann überschüssiges Volumen durch Abtupfen oder mittels einer Rakel entfernt werden. Hierdurch lässt sich eine unerwünschte Verschmutzung von Vorrichtungen zur Durchführung des Verfahrens durch das überschüssige Volumen vermeiden oder zumindest vermindern.

In weiterer Ausgestaltung des Verfahrens nach dem zweiten Aspekt der Erfindung umfasst das Verfahren einen Schritt E), der zeitlich nach Schritt C) durchgeführt wird und gemäß welchem ein Schließen der Schlitzanordnung durch eine elastische Rückstellwirkung des Materials des mit dem Hemm-Material wenigstens bereichsweise versehenen Funktionsteils erfolgt. Das Schließen der Schlitzanordnung erfordert somit keine aktive Maßnahme oder Vorrichtung.

Zweckmäßig kann durch das Schließen der Schlitzanordnung das Hemm-Material auf den Schlitzwandungsflächen, insbesondere vollflächig, verteilt werden. Das Schließen der Schlitzanordnung kann mit einer Wegnahme der Deformation einhergehen.

In weiterer Ausgestaltung des Verfahrens nach dem zweiten Aspekt der Erfindung wird zum Auswerfen des mit dem Hemm-Material wenigstens bereichsweise versehenen Funktionsteils zeitlich nach der Durchführung des Schritts E) die Einspannung des Funktionsteils gelöst und das Funktionsteil relativ zum Dorn infolge der Rückstellwirkung selbsttätig und/oder durch Verstellen des Dorns, insbesondere relativ zu einer Spannvorrichtung für die Einspannung, bewegt. Infolge des Auswerfens kann das Funktionsteil lose auf dem Dorn verbleiben oder vollständig von diesem abgleiten. Im ersten Fall kann vorteilhaft auf eine gesonderte Auswerfmaßnahme verzichtet werden. Im zweiten Fall lässt sich demgegenüber das Funktionsteil besonders kontrolliert entnehmen.

Die Schritte und Unterschritte der Verfahren nach den ersten beiden Erfindungsaspekten können in der alphabetischen und/oder numerischen Reihenfolge gemäß der voranstehenden Beschreibung durchgeführt werden. Sofern sinnhaft kann aber auch eine andere Reihenfolge gewählt werden.

Gemäß einem dritten Aspekt betrifft die Erfindung eine Vorrichtung für die Herstellung eines elastisch deformierbaren Funktionsteils, insbesondere eines Funktionsteil-Rohlings, für ein Medizinprodukt, insbesondere mittels eines Verfahrens gemäß dem ersten Aspekt der Erfindung wie oben beschrieben. Die Vorrichtung weist eine Spannvorrichtung zum Einspannen eines Rohlings für das Funktionsteil auf. Zudem weist die Vorrichtung eine, insbesondere hochglanzpolierte, Sonotrode auf, die zum Erzeugen eines Schlitzes der Schlitzanordnung in Schwingung mit einer Ultraschallfrequenz versetzbar und relativ zur Spanneinrichtung verstellbar ist. Die oben dargelegten Vorteile des Verfahrens gemäß dem ersten Aspekt der Erfindung lassen sich mittels der Vorrichtung ausnutzen, so dass besagte Vorteile sinngemäß auch für die Vorrichtung gemäß dem dritten Aspekt der Erfindung gelten.

Gemäß einem vierten Aspekt betrifft die Erfindung eine weitere Vorrichtung für die Herstellung eines elastisch deformierbaren Funktionsteils für ein Medizinprodukt, insbesondere mittels eines Verfahrens nach dem zweiten Aspekt der Erfindung wie oben beschrieben. Die Vorrichtung weist eine Spannvorrichtung zum Einspannen eines Funktionsteil-Rohlings auf. Ferner weist die Vorrichtung einen Dorn auf, wobei die Spannvorrichtung und der Dorn zum deformationsbedingten Öffnen oder Aufweiten der Schlitzanordnung des Funktionsteil-Rohlings relativ zueinander verstellbar sind. Außerdem weist die Vorrichtung eine, vorzugsweise einen Extruder und/oder eine Dosierpumpe umfassende, Dosiereinheit zum Austragen des Hemm-Materials auf, das die Selbstheilung des Materials des Funktionsteil-Rohlings, insbesondere im Bereich seiner Schlitzanordnung, hemmt oder verhindert, und zum, insbesondere bereichsweise oder vollflächigen, Auftragen des Hemm-Materials auf wenigstens eine, vorzugsweise auf zwei komplementäre, höchst vorzugsweise auf jede, der Schlitzwandungsflächen der Schlitzanordnung. Die Vorrichtung gemäß dem vierten Aspekt der Erfindung erlaubt es, die oben angeführten Vorteile des Verfahrens nach dem zweiten Aspekt der Erfindung auszunutzen, so dass besagte Vorteile sinngemäß auch für die Vorrichtung gemäß dem vierten Aspekt der Erfindung gelten.

Gemäß einem fünften Aspekt betrifft die Erfindung ein Medizinprodukt, insbesondere Silikonventileinrichtung, besonders bevorzugt Schlitzventileinrichtung, aufweisend wenigstens ein elastisch deformierbares, und vorzugsweise transparentes, Funktionsteil, das mittels eines Verfahrens gemäß dem ersten und/oder zweiten Aspekt der Erfindung hergestellt ist und das eine öffnungsfähige Schlitzanordnung aufweist, die sich bei einer elastischen Deformation des Funktionsteils aufweitet oder öffnet und bei Wegfall der Deformation wieder schließt, um einen durch das Medizinprodukt geführten Fluidstrom zu steuern. Die vorstehend erläuterten Vorteile des ersten, zweiten, dritten und vierten Erfindungsaspekts gelten sinngemäß für das Medizinprodukt gemäß dem fünften Aspekt der Erfindung.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist. Dabei beziehen sich gleiche Bezugszeichen auf gleiche oder ähnliche oder funktional gleiche Bauteile.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

### FIGURENKURZBESCHREIBUNG

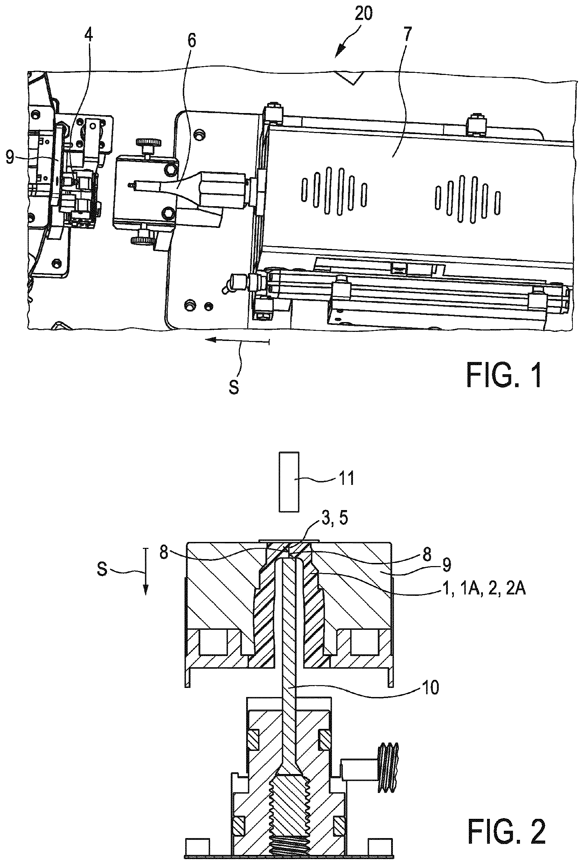

- Fig. 1: zeigt in schematischer Perspektivdarstellung eine Ausführungsform einer Vorrichtung gemäß einem dritten Aspekt der Erfindung für die Herstellung eines elastisch deformierbaren Funktionsteils für ein Medizinprodukt gemäß einem fünften Aspekt der Erfindung während der Durchführung eines Verfahrens nach einem ersten Aspekt der Erfindung,
- Fig. 2: in schematischer Schnittdarstellung eine Ausführungsform einer Vorrichtung gemäß einem vierten Aspekt der Erfindung für die Herstellung eines elastisch deformierbaren Funktionsteils für das Medizinprodukt gemäß dem fünften Aspekt der Erfindung während der Durchführung eines Verfahrens nach einem zweiten Aspekt der Erfindung,

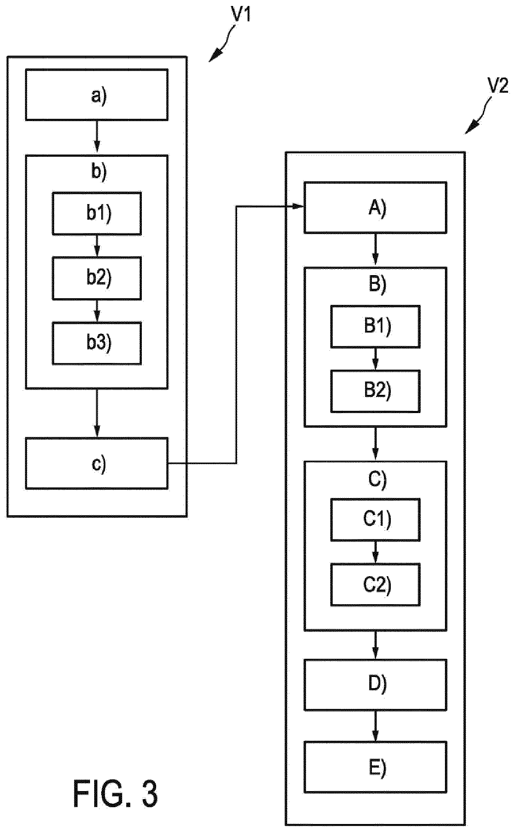

- Fig. 3: ein Ablaufschema für einen Durchführungsvorgang, in welchem die Verfahren gemäß den ersten beiden Aspekten der Erfindung zum Herstellen des elastisch deformierbaren Funktionsteils miteinander kombiniert werden.

### AUSFÜHRLICHE FIGURENBESCHREIBUNG

Die Fig. 1 zeigt schematisch eine Ausführungsform einer Vorrichtung 20 nach einem dritten Aspekt der Erfindung. Die Vorrichtung 20 dient der Herstellung eines elastisch deformierbaren Funktionsteils 1 für ein Medizinprodukt 2 gemäß einem fünften Aspekt der Erfindung. Vorliegend dient die Vorrichtung 20 der Herstellung eines elastisch deformierbaren Funktionsteil-Rohlings 1A.

Die Vorrichtung 20 umfasst eine Spanneinrichtung 9 zum Einspannen eines Rohlings 4 für das Funktionsteil 1. Zudem weist die Vorrichtung 20 eine Sonotrode 6 auf. Die Sonotrode 6 kann mittels eines Ultraschallgenerators 7 der Vorrichtung 20 in Schwingung mit einer Ultraschallfrequenz versetzt werden. Die Sonotrode 6 ist relativ zur Spanneinrichtung 9 verstellbar. In Fig. 1 ist die Vorrichtung 20 während der Durchführung eines Verfahrens V1 gemäß einem ersten Aspekt der Erfindung gezeigt.

Fig. 2 zeigt schematisch eine Ausführungsform einer Vorrichtung 30 gemäß einem vierten Aspekt der Erfindung. Die Vorrichtung 30 dient der Herstellung eines elastisch deformierbaren Funktionsteils 1 für ein gemäß dem fünften Aspekt der Erfindung ausgeführtes Medizinprodukt 2. Die Vorrichtung 30 umfasst eine Spanneinrichtung 9 zum Einspannen eines Funktionsteil-Rohlings 1A. Bei dem Funktionsteil-Rohling 1A für die Vorrichtung 30 kann es sich um das Funktionsteil 1 handeln, das zuvor mittels der Vorrichtung 20 gemäß dem Verfahren V1 hergestellt wurde. Mit anderen Worten: Das Produkt des Verfahrens V1 kann in Form eines Rohlings als eines der Edukte für ein Verfahren V2 gemäß einem zweiten Aspekt der Erfindung verwendet werden, während dessen Durchführung die Vorrichtung 30 in der Fig. 2 gezeigt ist.

Die Vorrichtung 30 weist einen Dorn 10 auf. Der Dorn 10 und die Spanneinrichtung 9 sind relativ zueinander verstellbar. Ferner umfasst die Vorrichtung 30 eine Dosiereinheit 11 zum Austragen eines Hemm-Materials. Das Hemm-Material hemmt oder verhindert die Selbstheilung eines Materials des Funktionsteil-Rohlings 1A. Insbesondere kann mittels des Hemm-Materials eine Selbstheilung im Bereich einer Schlitzanordnung 3 des Funktionsteil-Rohlings 1A gehemmt oder verhindert werden. Die Dosiereinheit 11 kann einen Extruder und/oder eine Dosierpumpe aufweisen, mittels welcher das Hemm-Material gefördert und dosiert werden kann.

In der Fig. 3 ist ein Aufbauschema oder Ablaufdiagramm eines Durchführungsvorgangs bei Durchführung der beiden Verfahren V1, V2 nacheinander gezeigt. Der Durchführungsvorgang lässt sich also selbst als kombiniertes Verfahren verstehen, das die beiden Verfahren V1, V2 als zeitlich aufeinanderfolgende Schritte umfasst und mittels welchem das elastisch deformierbare Funktionsteil 1 hergestellt werden kann.

Das Verfahren V1 dient einem Herstellen eines elastisch deformierbaren Funktionsteils 1 für ein Medizinprodukt 2. Das mittels des Verfahrens V1 hergestellte Funktionsteil 1 kann als Funktionsteil-Rohling 1A für das Verfahren V2 verwendet werden. Das Funktionsteil 1 weist eine öffnungsfähige Schlitzanordnung 3 auf. Die Schlitzanordnung 3 weitet sich bei einer elastischen Deformation des Funktionsteils 1 auf oder öffnet sich bei elastischer Deformation des Funktionsteils 1. Bei Wegfall der Deformation schließt sich die Schlitzanordnung 3 wieder. Wie bereits angesprochen, kann das mittels des Verfahrens V1 herzustellende elastisch deformierbare Funktionsteil 1 als Funktionsteil-Rohling 1A dienen, welcher dann gemäß dem Verfahren V2 weiterverarbeitet wird, um das Funktionsteil 1 fertigzustellen. Die vorstehend genannten Eigenschaften des mittels des Verfahrens V1 hergestellten Funktionsteils 1 sind folglich auch bei dem Funktionsteil-Rohling 1A für das Verfahren V2 vorhanden.

Das Verfahren V1 umfasst einen Schritt a), gemäß welchem ein Rohling 4 für das Funktionsteil 1 bereitgestellt wird. Der Rohling 4 weist ein elastisches Material auf. Dieses Material kann transparent ausgebildet sein. Der Rohling 4 kann aus dem elastischen, und insbesondere transparenten, Material bestehen. Das Verfahren V1 weist ferner einen Schritt b) auf, gemäß welchem das Material des Rohlings 4 wenigstens bereichsweise durchtrennt wird. Dabei erfolgt das Durchtrennen des Materials des Rohlings 4 gemäß dem Schritt b) mittels Ultraschalls, so dass wenigstens ein Schlitz 5 der Schlitzanordnung 3 des Funktionsteils 1 erzeugt wird. Die Schlitzanordnung 3 kann mehrere Schlitze 5 umfassen. Derartige mehrere Schlitze 5 können beispielsweise in Kreuz- oder Sternanordnung angeordnet sein. Der wenigstens eine Schlitz 5 kann geradlinig oder gekrümmt geformt werden.

Das Material des Rohlings 4 kann ein Elastomer aufweisen oder aus einem Elastomer bestehen. Vorzugsweise ist das Elastomer ein thermoplastisches Elastomer. Das Elastomer kann insbesondere ausgewählt sein aus einer Gruppe bestehend aus Silikonelastomer, Silikonkautschuk, Acrylnitril-Butadien-Kautschuk, hydrierter Acrylnitril-Butadien-Kautschuk, Styrol-Butadien-Kautschuk und Mischungen von wenigstens zwei der vorgenannten Elastomere. Alternativ oder in Kombination kann es sich bei dem Material des Rohlings 4 um ein anderes Polymer handeln, das vernetzungsfähig ist oder in dem Vernetzungsreaktionen überwiegen.

Gemäß Fig. 3 umfasst der Schritt b) des Verfahrens V1 einen Unterschritt b1). Gemäß dem Unterschritt b1) wird die Sonotrode 6 aktiviert, so dass sie in eine Schwingung mit Ultraschallfrequenz versetzt wird. Die Ultraschallfrequenz kann 20 kHz bis 40 kHz, vorzugsweise 30 kHz bis 38 kHz, betragen. Die Ultraschallfrequenz stimmt vorzugsweise mit einer Resonanzfrequenz der Sonotrode 6 überein. Die Sonotrode 6 kann hochglanzpoliert sein. Die Sonotrode 6 kann mit oder aus einem metallischen Werkstoff, insbesondere einer Legierung mit Eisen und/oder Titan und/oder Aluminium, ausgebildet sein. Die Sonotrode 6 wird wie in Fig. 1 erkennbar von einem Ultraschallgenerator 7 gespeist. Dabei versetzt der Ultraschallgenerator 7 die Sonotrode 6 in Unterschritt b1) in Schwingung mit Ultraschallfrequenz. Gemäß Fig. 3 umfasst der Schritt b) des Verfahrens V1 zudem einen Unterschritt b2). Gemäß dem Unterschritt b2) erfolgt ein Verstellen der Sonotrode 6 und/oder des Rohlings 4 relativ zueinander in einer Schlitzrichtung S. Infolge des Verstellens gemäß Unterschritt b2) wird das Material des Rohlings 4 wenigstens bereichsweise durchtrennt, um den wenigstens einen Schlitz 5 zu erzeugen. Das Verstellen gemäß Unterschritt b2) kann mit einer Vorschubgeschwindigkeit von 0,02 m/s bis 0,2 m/s, vorzugsweise 0,05 bis 0,15 m/s, erfolgen.

Der Schritt b) des Verfahrens V1 weist gemäß Fig. 3 ferner einen Unterschritt b3) auf. Gemäß dem Unterschritt b3) erfolgt ein Verweilen der Sonotrode 6 in einer vorbestimmten Endposition für eine vorbestimmte Verweildauer, wenn die Sonotrode 6 bei der Durchführung des Unterschritts b2) die vorbestimmte Endposition relativ zum Rohling 4 erreicht hat. In der Endposition kann die Sonotrode 6 den Schlitz 5 vollständig durchragen. Beispielsweise durchragt die Sonotrode 6 in ihrer Endposition den Schlitz 5 entlang der Schlitzrichtung S vollständig. Die Verweildauer kann 50 ms bis 500 ms, vorzugsweise 80 ms bis 300 ms, betragen.

Eine durch das wenigstens bereichsweise Durchtrennen des Materials des Rohlings 4 gemäß Schritt b) erreichbare Schnittqualität des erzeugten Schlitzes 5 kann durch Verfahrensparameter wie der gewählten Ultraschallfrequenz der Sonotrode 6, der Vorschubgeschwindigkeit und der Verweildauer gezielt beeinflusst werden. Die Schnittqualität lässt sich entsprechend beeinflussen, um eine Fluiddichtheit der Schlitzanordnung 3 zu verbessern. Zudem lässt sich durch gezieltes Beeinflussen der Schnittqualität eine Haftung des Hemm-Materials verbessern, das eine Selbstheilung des Materials des Funktionsteils 1, 1A, insbesondere im Bereich seiner Schlitzanordnung 3, hemmt oder verhindert. Durch Abstimmung der Verfahrensparameter lässt sich vorteilhafterweise eine Rauigkeit von Schlitzwandungsflächen 8 des Schlitzes 5 gezielt beeinflussen.

Wenn die Sonotrode 6 aktiviert ist, wird sie in Ultraschallschwingung mit einer Amplitude versetzt. Wird die Sonotrode 6 deaktiviert, so ist keine Amplitude vorhanden. Die aktivierte Sonotrode 6 kann mit 50 % bis 100 %, vorzugsweise 60 % bis 90%, ihrer Amplitudenleistung schwingen.

Beispielsweise wird zum Abschluss der Durchführung des Schritts b) des Verfahrens V1 die Sonotrode 6 deaktiviert, so dass die Schwingung der Sonotrode 6 angehalten wird. Infolge des Deaktivierens der Sonotrode 6 kann also die Sonotrode 6 aufhören zu schwingen.

Das Verfahren V1 umfasst gemäß Fig. 3 zudem einen zusätzlichen Schritt c), gemäß welchem die Sonotrode 6 und/oder das geschlitzte Funktionsteil 1 voneinander entfernt werden. Das Deaktivieren der Sonotrode 6 kann zeitlich vor, während oder nach dem Verweilen in der Endposition vorgenommen werden. Vorzugsweise ist die Sonotrode 6 beim Entfernen gemäß Schritt c) bereits deaktiviert.

Wie bereits erwähnt, zeigt Fig. 3 zudem den Ablauf eines Verfahrens V2 gemäß einem weiteren Aspekt der Erfindung. Das Verfahren V2 dient dem Herstellen eines elastisch deformierbaren Funktionsteils 1 für das Medizinprodukt 2 gemäß dem fünften Aspekt der Erfindung. Das Funktionsteil 1 weist eine öffnungsfähige Schlitzanordnung 3 auf, die sich bei einer elastischen Deformation des Funktionsteils 1 aufweitet oder öffnet und die sich bei Wegfall der Deformation wieder schließt. Das Verfahren V2 weist einen Schritt A) auf, gemäß welchem ein geschlitzter Funktionsteil-Rohling 1A bereitgestellt wird.

Der Funktionsteil-Rohling 1A ist beispielsweise gemäß dem vorstehend beschriebenen Verfahren V1 hergestellt. Bei dem Funktionsteil-Rohling 1A handelt es sich somit vorliegend - wie oben bereits erwähnt - beispielsweise um das mittels des Verfahrens V1 hergestellte Funktionsteil 1. Der Funktionsteil-Rohling 1A weist entsprechend eine Schlitzanordnung 3 auf, die wenigstens einen Schlitz 5 umfasst. An dem wenigstens einen Schlitz 5 ist ein elastisches, und insbesondere transparentes, Material des Funktionsteil-Rohlings 1A wenigstens bereichsweise durchtrennt. Der wenigstens eine Schlitz 5 ist beispielsweise gemäß Schritt b) des Verfahrens V1 erzeugt.

Das Verfahren V2 umfasst außerdem einen Schritt B). Gemäß dem Schritt B) wird die Schlitzanordnung 3 des Funktionsteil-Rohlings 1A aufgeweitet oder geöffnet. Zudem weist das Verfahren V2 einen Schritt C) auf, gemäß welchem wenigstens eine Schlitzwandungsfläche 8 der Schlitzanordnung 3 des Funktionsteil-Rohlings 1A mit dem Hemm-Material versehen wird. Das Hemm-Material hemmt oder verhindert eine Selbstheilung des Materials des Funktionsteil-Rohlings 1A, insbesondere im Bereich seiner Schlitzanordnung 3. Das Versehen der Schlitzwandungsfläche 8 kann durch Beschichten oder Beimpfen mit dem Hemm-Material erfolgen. Beispielsweise werden zwei komplementäre Schlitzwandungsflächen 8 der Schlitzanordnung 3, die gemeinsam den wenigstens einen Schlitz 5 begrenzen, mit dem Hemm-Material versehen. Vorzugsweise werden alle Schlitzwandungsflächen 8 der Schlitzanordnung 3 mit dem Hemm-Material versehen.

Für einen Übergang zwischen dem Verfahren V1 und dem Verfahren V2 kann der Funktionsteil-Rohling 1A von einer Spannvorrichtung 9 der Vorrichtung 20 in eine Spannvorrichtung 9 der Vorrichtung 30 versetzt werden. Alternativ ist es auch denkbar, dass ein- und dieselbe Spannvorrichtung 9 verwendet wird, wenn die Vorrichtungen 20, 30 in einer gemeinsamen Anlage vorgesehen sind. Beim Umsetzen des Funktionsteil-Rohlings zwischen den Spannvorrichtungen 9 der Vorrichtungen 20, 30 wird vorzugsweise eine Ausrichtung der Schlitzanordnung 3 beibehalten.

Das Hemm-Material kann ein Öl aufweisen. Beispielsweise kann das Öl ausgewählt sein aus einer Gruppe bestehend aus Silikonöl, alkyliertem Naphthalin (AN), Chlortrifluoroethylen (CTFE), Esteröl, Mineralöl, insbesondere synthetisches Mineralöl, multialkyliertes Cyclopentan (MAC), Polyalphaolefin (PAO), Polyphenylether (PPE), Polyglykolöl (PG), perfluoriertes Polyetheröl (PFPE), Triglyceride und Mischungen von wenigstens zwei der vorgenannten Öle. Bei dem Öl kann es sich insbesondere um ein fluoriertes Silikonöl handeln.

Das Hemm-Material kann zudem einen Verdicker aufweisen. Der Verdicker kann ausgewählt sein aus einer Gruppe bestehend aus Aluminiumseife, Aluminiumkomplexseife, Bariumseife, Bariumkomplexseife, Calciumseife, Calciumkomplexseife, Lithiumseife, Lithiumkomplexseife, Natriumseife, Natriumkomplexseife, Polytetrafluorethylen (PTFE), anorganischem Verdicker, insbesondere Bentonit, Polyharnstoff, Silica und Mischungen von wenigstens zwei der vorgenannten Verdicker.

Das Hemm-Material kann außerdem einen Feststoff aufweisen, der kein Verdicker ist, wobei der Feststoff insbesondere ausgewählt ist aus einer Gruppe bestehend aus Salzen, Sacchariden, Vitaminen und Mischungen von wenigstens zwei der vorgenannten Feststoffe.

Das Hemm-Material kann flüssig sein. Das Hemm-Material kann zähflüssig oder pastös sein. Das Hemm-Material kann thixotrop oder antithixotrop sein.

Gemäß Fig. 3 umfasst der Schritt B) des Verfahrens V2 einen Unterschritt B1). Gemäß dem Unterschritt B1) wird der geschlitzte Funktionsteil-Rohling 1A eingespannt. Beispielsweise wird der geschlitzte Funktionsteil-Rohling 1A mittels einer Spannrichtung 9 eingespannt, die der Vorrichtung 30 zugeordnet ist. Der Schritt B) umfasst vorliegend außerdem einen Unterschritt B2), gemäß welchem der Funktionsteil-Rohling 1A deformiert wird. Infolge des Deformierens des Funktionsteil-Rohlings 1A gemäß Unterschritt B2) wird die Schlitzanordnung 3 des Funktionsteil-Rohlings 1A geöffnet oder aufgeweitet.

Zeitlich vor der Durchführung des Unterschritts B1) und/oder des Unterschritts B2) kann der Funktionsteil-Rohling 1A an oder auf einem Dorn 10 angeordnet werden. Dies ist in der Fig. 2 in der Art einer Momentaufnahme gezeigt. Beispielsweise kann der Funktionsteil-Rohling 1A deformationsfrei an oder auf dem Dorn 10 angeordnet werden. Während der Durchführung des Unterschritts B2) - also beim Deformieren des Funktionsteil-Rohlings 1A - können der Dorn 10 und/oder der Funktionsteil-Rohling 1A relativ zueinander verstellt werden, so dass der Funktionsteil-Rohling 1A mittels des Dorns 10 deformiert wird. Der Dorn 10 kann in einer Bohrung des Funktionsteil-Rohlings 1A eingeführt werden. Diese Bohrung kann wie in Fig. 2 erkennbar einenends geöffnet und anderenends von einem integralen Membranabschnitt des Funktionsteil-Rohlings 1A verschlossen sein. Dabei kann die Schlitzanordnung 3 an dem Membranabschnitt vorhanden sein. Der Dorn 10 kann entlang der Schlitzrichtung S auf den Membranabschnitt wirken. Hierdurch kann die Schlitzanordnung 3 infolge der Deformierung des Funktionsteil-Rohlings 1A aufgedrückt werden. Die Schlitzanordnung 3 kann infolge des Deformierens aufklaffen.

Der Schritt C) des Verfahrens V2 umfasst gemäß Fig. 3 einen Unterschritt C1). Gemäß dem Unterschritt C1) erfolgt ein gegenseitiges Annähern des Funktionsteil-Rohlings 1A und einer Dosiereinheit 11. Die Dosiereinheit 11 kann der Vorrichtung 30 zugeordnet sein, vgl. Fig. 2. Die Dosiereinheit 11 dient zum Austragen des Hemm-Materials. Das gegenseitige Annähern gemäß Unterschritt C1) erfolgt, bis ein vorbestimmter Soll-Auftragsabstand erreicht wird. Das gegenseitige Annähern gemäß Unterschritt C1) kann durch Verstellen von Funktionsteil-Rohling 1A und/oder Dosiereinheit 11 relativ zueinander erfolgen. Der Soll-Auftragsabstand kann null sein. Der Soll-Auftragsabstand kann kleiner null sein, d. h. die Dosiereinheit 11 kann in diesem Fall teilweise in die Schlitzanordnung 3 hineinragen. Der Soll-Auftragsabstand kann - 0,4 mm bis + 0,2 mm, vorzugsweise - 0,2 mm bis + 0,1 mm, betragen.

Der Schritt C) umfasst beispielsweise einen weiteren Unterschritt C2). Gemäß dem Unterschritt C2) wird, wenn der Soll-Auftragsabstand erreicht ist, das Hemm-Material ausgetragen, um das Hemm-Material wenigstens bereichsweise auf die wenigstens eine der Schlitzwandungsflächen 8 aufzutragen. Vorzugsweise wird das Hemm-Material dabei vollflächig auf die wenigstens eine Schlitzwandungsfläche 8 aufgetragen. Beispielsweise kann das Hemm-Material auf zwei komplementäre der Schlitzwandungsflächen 8 aufgetragen werden, die gemeinsam den Schlitz 5 der Schlitzanordnung 3 begrenzen. Das Hemm-Material kann in Unterschritt C2) auf jede der Schlitzwandungsflächen 8 der Schlitzanordnung 3 aufgetragen werden.

Gemäß Fig. 3 umfasst das Verfahren V2 außerdem einen zusätzlichen Schritt D). Gemäß dem Schritt D) wird ein überschüssiges Volumen des Hemm-Materials wenigstens teilweise entfernt. Dabei wird das überschüssige Volumen von einem die wenigstens eine Schlitzwandungsfläche 8 wenigstens bereichsweise benetzenden Auftragsvolumen des Hemm-Materials abgetrennt. Das wenigstens teilweise Entfernen des überschüssigen Volumens des Hemm-Materials gemäß Schritt D) kann beispielsweise durch Umkehr einer Pumprichtung oder Extrusionsrichtung oder Förderrichtung der Dosiereinheit 11 erfolgen. Alternativ oder zusätzlich kann die Dosiereinheit zum Entfernen des überschüssigen Volumens relativ zum Funktionsteil-Rohling verstellt werden. Hierdurch lässt sich ein wenigstens teilweises "Zurückziehen" einer ausgetragenen Menge des Hemm-Materials erreichen. Beispielsweise kann eine ausgetragene Raupe des Hemm-Materials teilweise zurückgezogen werden. Alternativ oder zusätzlich kann überschüssiges Volumen des Hemm-Materials durch Abtupfen oder mittels einer Rakel entfernt werden. Zum Entfernen des überschüssigen Volumens mittel der Rakel kann dieselbe entlang einer Erstreckung des wenigstens einen Schlitzes 5 relativ zum Funktionsteil 1 verstellt werden.

Mittels einer Kamera können eine Geometrie der Schlitzanordnung 3 und/oder ein wünschenswerterweise vollständiges Benetzen der wenigstens einen Schlitzwandungsfläche 8 optisch kontrolliert werden.

Das Verfahren V2 gemäß Fig. 3 umfasst außerdem einen zusätzlichen Schritt E), der zeitlich nach Schritt C) durchgeführt wird. Gemäß dem Schritt E) wird die Schlitzanordnung 3 infolge einer elastischen Rückstellwirkung des Materials des beschichteten Funktionsteils 1 geschlossen. Durch das Schließen gemäß Schritt E) kann das Hemm-Material auf den Schlitzwandungsflächen 8 verteilt werden. Das Schließen der Schlitzanordnung 3 durch die elastische Rückstellwirkung geht mit Wegnahme der Deformation einher.

Beispielsweise wird zum Auswerfen des mit dem Hemm-Material wenigstens bereichsweise versehenen Funktionsteils 1 zeitlich nach der Durchführung des Schritts E) die Einspannung des Funktionsteils 1 gelöst. Das Funktionsteil 1 wird dann relativ zum Dorn 10 infolge der Rückstellwirkung selbsttätig bewegt. Alternativ oder zusätzlich wird das Funktionsteil 1 durch Verstellen des Dorns 10, insbesondere relativ zur Spanneinrichtung 9, bewegt. Im Anschluss kann das fertiggestellte Funktionsteil 1 entnommen werden. Der Dorn 10 kann nach Entnahme des Funktionsteils 1 in seine Ausgangsposition zurückverstellt werden, so dass er für eine erneute Durchführung des Verfahrens V2 verfügbar ist. Das Entnehmen des fertiggestellten Funktionsteils 1 kann durch ein Auswerfen aus der Vorrichtung 30 erfolgen.

Die Sonotrode 6 der Vorrichtung 20 ist zum Erzeugen des wenigstens einen Schlitzes 5 der Schlitzanordnung 3 in Schwingung mit Ultraschallfrequenz versetzbar.

Der Dorn 10 der Vorrichtung 30 dient dem deformationsbedingten Öffnen oder Aufweiten der Schlitzanordnung 3 des Funktionsteil-Rohlings 1A. Die Dosiereinheit 11 zum Austragen des Hemm-Materials der Vorrichtung 30 dient dem, insbesondere bereichsweisen oder vollflächigen, Auftragen des Hemm-Materials auf wenigstens einer, vorzugsweise auf zwei komplementären, höchst vorzugsweise auf jeder, der Schlitzwandungsflächen 8 der Schlitzanordnung 3.

Das in Fig. 2 erkennbare Funktionsteil 1 ist Teil eines Medizinprodukts 2 gemäß dem fünften Aspekt der Erfindung. Beispielsweise handelt es sich bei dem Medizinprodukt 2 um eine Silikonventileinrichtung 2A. Die Silikonventileinrichtung 2A kann als Schlitzventileinrichtung ausgebildet sein. Durch das deformationsbedingte Aufweiten bzw. Öffnen und Schließen des Funktionsteils 1 kann ein durch das Medizinprodukt 2 geführter Fluidstrom gesteuert werden. Das mittels des Verfahrens V1 hergestellte Funktionsteil 1, welches als Funktionsteil-Rohling 1A für das Verfahren V2 verwendet werden kann, unterscheidet sich von dem mittels des Verfahrens V2 hergestellten Funktionsteil 1 ausschließlich darin, dass bei Letzterem die wenigstens eine Schlitzwandungsfläche 8 mit dem Hemm-Material versehen ist.

Das Medizinprodukt 2 kann als Verbindungseinrichtung eines medizinischen Infusionssystems oder für ein medizinisches Infusionssystem verwendet werden. Entsprechend kann das Medizinprodukt 2 bzw. die Silikonventileinrichtung 2A als Dreiwegehahn gestaltet sein. Alternativ kann das Medizinprodukt 2 bzw. die Silikonventileinrichtung 2A als Einwege- oder Zweiwegehahn oder als Hahnbank, d.h. als eine Einheit oder ein System mit oder aus in Reihe, d.h. hintereinander, geschalteten Einwegehähnen gestaltet sein. Das Funktionsteil 1, 1A des Medizinprodukts 2 kann einen Ventilkörper des Medizinprodukts 2 ausbilden. Der Ventilkörper kann becher- oder glockenförmig gestaltet sein. Ein Boden des becher- oder glockenförmigen Ventilkörpers kann durch einen integralen Membranabschnitt gebildet sein, an welchem die Schlitzanordnung 3 vorhanden ist. Der Ventilkörper kann einstückig ausgebildet sein. Die Schlitzwandungen 8 liegen in einem deformationsfreien Zustand des Ventilkörpers beispielsweise aneinander an, so dass ein durch das Medizinprodukt 2 führender Fluidpfad fluiddicht verschlossen ist. Bei deformationsbedingtem Aufweiten oder Öffnen der Schlitzanordnung 3 wird dieser Fluidpfad freigegeben, so dass er von einem Fluid durch die Schlitzanordnung 3 hindurch durchströmbar ist. Beispielweise kann die Schlitzanordnung aufgeweitet oder geöffnet werden, indem eine Anschlusskomponente unter Deformation des Funktionsteils 1 an das Medizinprodukt 2 und an den durch das Medizinprodukt 2 geführten Fluidpfad fluidleitend angeschlossen wird.

## Patentansprüche

1. Verfahren (V1) zum Herstellen eines elastisch deformierbaren Funktionsteils (1), insbesondere Funktionsteil-Rohlings (1A), für ein Medizinprodukt (2), wobei das Funktionsteil (1, 1A) eine öffnungsfähige Schlitzanordnung (3) aufweist, die sich bei einer elastischen Deformation des Funktionsteils (1) aufweitet oder öffnet und bei Wegfall der Deformation wieder schließt, umfassend nachfolgende Schritte:
a) Bereitstellen eines Rohlings (4) für das Funktionsteil (1, 1A), der ein elastisches, und insbesondere transparentes, Material aufweist oder aus einem elastischen, und insbesondere transparenten, Material besteht,
b) wenigstens bereichsweises Durchtrennen des Materials des Rohlings (4) mittels Ultraschalls, so dass wenigstens ein Schlitz (5) der Schlitzanordnung (3) des Funktionsteils (1, 1A) erzeugt wird.

2. Verfahren (V1) nach Anspruch 1, **dadurch gekennzeichnet, dass** der Schritt b) folgenden Unterschritt umfasst:
b1) Aktivieren einer Sonotrode (6), so dass die Sonotrode (6) in eine Schwingung mit Ultraschallfrequenz versetzt wird.

3. Verfahren (V1) nach Anspruch 1 oder 2, **dadurch gekennzeichnet, dass** der Schritt b) folgenden Unterschritt umfasst:
b2) Verstellen der Sonotrode (6) und/oder des Rohlings (4) relativ zueinander in einer Schlitzrichtung (S), um das Material des Rohlings (4) wenigstens bereichsweise zu durchtrennen und den wenigstens einen Schlitz (5) zu erzeugen.

4. Verfahren (V1) nach Anspruch 3, **dadurch gekennzeichnet, dass** der Schritt b) folgenden zusätzlichen Unterschritt umfasst:
b3) Verweilen der Sonotrode (6) in einer vorbestimmten Endposition für eine vorbestimmte Verweildauer, wenn die Sonotrode (6) bei der Durchführung des Unterschritts b2) die vorbestimmte Endposition relativ zum Rohling (4) erreicht hat.

5. Verfahren (V1) nach einem der vorhergehenden Ansprüche, **dadurch gekennzeichnet, dass** zum Abschluss der Durchführung des Schritts b) die Sonotrode (6) deaktiviert wird, so dass die Schwingung der Sonotrode (6) angehalten wird.

6. Verfahren (V1) nach einem der vorhergehenden Ansprüche, **dadurch gekennzeichnet, dass**
das Verfahren (V1) zusätzlich folgenden Schritt umfasst:
c) Entfernen der Sonotrode (6) und/oder des geschlitzten Funktionsteils (1, 1A) voneinander.

7. Verfahren (V2) zum Herstellen eines elastisch deformierbaren Funktionsteils (1) für ein Medizinprodukt (2), wobei das Funktionsteil (1) eine öffnungsfähige Schlitzanordnung (3) aufweist, die sich bei einer elastischen Deformation des Funktionsteils (1) aufweitet oder öffnet und bei Wegfall der Deformation wieder schließt, umfassend nachfolgende Schritte:
A) Bereitstellen eines geschlitzten Funktionsteil-Rohlings (1A) mit einer Schlitzanordnung (3), die wenigstens einen Schlitz (5) aufweist, an welchem ein elastisches, und insbesondere transparentes, Material des Funktionsteil-Rohlings (1A) wenigstens bereichsweise mittels Ultraschalls durchtrennt ist und der vorzugsweise gemäß einem Verfahren (V1) nach einem der vorhergehenden Ansprüche erzeugt ist,
B) Aufweiten oder Öffnen der Schlitzanordnung (3),
C) wenigsten bereichsweises, vorzugsweise vollflächiges, Versehen wenigstens einer Schlitzwandungsfläche (8) der Schlitzanordnung (3) des Funktionsteil-Rohlings (1A) mit einem Hemm-Material, das eine Selbstheilung des Materials des Funktionsteil-Rohlings (1A), insbesondere im Bereich seiner Schlitzanordnung (3), hemmt oder verhindert.

8. Verfahren (V2) nach Anspruch 7, **dadurch gekennzeichnet, dass** der Schritt B) folgende Unterschritte umfasst:
B1) Einspannen des geschlitzten Funktionsteil-Rohlings (1A),
B2) Deformieren des Funktionsteil-Rohlings (1A), um die Schlitzanordnung (3) des Funktionsteil-Rohlings (1A) zu öffnen oder aufzuweiten.

9. Verfahren (V2) nach Anspruch 8, **dadurch gekennzeichnet, dass** zeitlich vor Durchführung des Unterschritts B1) und/oder des Unterschritts B2) der Funktionsteil-Rohling (1A), insbesondere deformationsfrei, an oder auf einem Dorn (10) angeordnet wird, und/oder dass während der Durchführung des Unterschritts B2) der Dorn (10) und/oder der Funktionsteil-Rohling (1A) relativ zueinander verstellt werden, so dass der Funktionsteil-Rohling (1A) mittels des Dorns (10) deformiert wird.

10. Verfahren (V2) nach einem der Ansprüche 7 bis 9, **dadurch gekennzeichnet, dass** der Schritt C) folgende Unterschritte umfasst:
C1) Gegenseitiges Annähern des Funktionsteil-Rohlings (1A) und einer Dosiereinheit (11) zum Austragen des Hemm-Materials, bis ein vorbestimmter Soll-Auftragsabstand erreicht wird,
C2) wenn der Soll-Auftragsabstand erreicht ist: Austragen des Hemm-Materials, um das Hemm-Material wenigstens bereichsweise, vorzugsweise vollflächig, auf die wenigstens eine Schlitzwandungsfläche (8) aufzutragen.

11. Verfahren (V2) nach einem der Ansprüche 7 bis 10, **dadurch gekennzeichnet, dass** das Verfahren (V2) folgenden zusätzlichen Schritt umfasst:
D) Wenigstens teilweises Entfernen eines überschüssigen Volumens des Hemm-Materials, um das überschüssige Volumen von einem die wenigstens eine Schlitzwandungsfläche (8) wenigstens bereichsweise benetzenden Auftragsvolumen des Hemm-Materials abzutrennen.

12. Verfahren (V2) nach einem der Ansprüche 7 bis 11, **dadurch gekennzeichnet, dass** das Verfahren (V2) folgenden zusätzlichen Schritt umfasst, der zeitlich nach Schritt C) durchgeführt wird:
E) Schließen der Schlitzanordnung (8) durch eine elastische Rückstellwirkung des Materials des mit dem Hemm-Material wenigstens bereichsweise versehenen Funktionsteils (1).

13. Verfahren (V2) nach Anspruch 12, bei Rückbezug auf Anspruch 9, **dadurch gekennzeichnet, dass** zum Auswerfen des beschichteten Funktionsteils (1) zeitlich nach der Durchführung des Schritts E) die Einspannung des Funktionsteils (1) gelöst und das Funktionsteil (1) relativ zum Dorn (10) infolge der Rückstellwirkung selbsttätig und/oder durch Verstellen des Dorns (10), insbesondere relativ zu einer Spannvorrichtung (9) für die Einspannung, bewegt wird.

14. Vorrichtung (20) für die Herstellung eines elastisch deformierbaren Funktionsteils (1), insbesondere eines Funktionsteil-Rohlings (1A), für ein Medizinprodukt (2) mittels eines Verfahrens (V1) nach einem der Ansprüche 1 bis 6, aufweisend
eine Spannvorrichtung (9) zum Einspannen eines Rohlings (4) für das Funktionsteil (1, 1A), und
eine Sonotrode (6), die zum Erzeugen eines Schlitzes (5) der Schlitzanordnung (3) in Schwingung mit einer Ultraschallfrequenz versetzbar und relativ zur Spannvorrichtung (9) verstellbar ist.

15. Vorrichtung (30) für die Herstellung eines elastisch deformierbaren Funktionsteils (1) für ein Medizinprodukt (2) mittels eines Verfahrens (V2) nach einem der Ansprüche 7 bis 13, aufweisend
eine Spannvorrichtung (9) zum Einspannen eines Funktionsteil-Rohlings (1A), einen Dorn (10), wobei die Spannvorrichtung (9) und der Dorn (10) zum deformationsbedingten Öffnen oder Aufweiten der Schlitzanordnung (3) des Funktionsteil-Rohlings (1A) relativ zueinander verstellbar sind, und
eine, vorzugsweise einen Extruder und/oder eine Dosierpumpe aufweisende, Dosiereinheit (11) zum Austragen des Hemm-Materials, das die Selbstheilung des Materials des Funktionsteil-Rohlings (1A), insbesondere im Bereich seiner Schlitzanordnung (3), hemmt oder verhindert, und zum, insbesondere bereichsweisen oder vollflächigen, Auftragen des Hemm-Materials auf wenigstens eine der Schlitzwandungsflächen (8) der Schlitzanordnung (3).

16. Medizinprodukt (2), insbesondere Silikonventileinrichtung (2A), aufweisend wenigstens ein elastisch deformierbares, und vorzugsweise transparentes, Funktionsteil (1, 1A), das mittels eines Verfahrens (V1, V2) nach einem der Ansprüche 1 bis 13 hergestellt ist und das eine öffnungsfähige Schlitzanordnung (3) aufweist, die sich bei einer elastischen Deformation des Funktionsteils (1, 1A) aufweitet oder öffnet und bei Wegfall der Deformation wieder schließt, um einen durch das Medizinprodukt (2) geführten Fluidstrom zu steuern.
